(19) Europäisches Patentamt
European Patent Office
Office européen des brevets

(11) Veröffentlichungsnummer : **0 022 460**
**B1**

(12) # EUROPÄISCHE PATENTSCHRIFT

(45) Veröffentlichungstag der Patentschrift : **24.11.82**

(21) Anmeldenummer : **80102703.8**

(22) Anmeldetag : **16.05.80**

(51) Int. Cl.³ : **C 07 C 43/115**, C 07 C 43/162, A 61 K 7/46, C 11 B 9/00// C07C41/01

(54) **Aliphatische Ether des Hydroximethylcyclododecans und ihre Verwendung zur Herstellung von Riechstoffkompositionen.**

(30) Priorität : **13.07.79 DE 2928347**

(43) Veröffentlichungstag der Anmeldung : **21.01.81 (Patentblatt 81/03)**

(45) Bekanntmachung des Hinweises auf die Patenterteilung : **24.11.82 Patentblatt 82/47**

(84) Benannte Vertragsstaaten : **CH FR GB IT LI**

(56) Entgegenhaltungen :
**DE B 1 211 174**
**DE B 2 152 016**

**Chemical Abstracts Band 80, Nr. 17, 29. April 1974 Columbus, Ohio, USA E. KLEIN et al. « Prins reaction with cyclic olefins. I. Reaction of cyclododecene with paraformaldehyde » Seite 385, Spalte 1, Abstract Nr. 95630u in Verbindung mit Chemical Abstracts Subject Index Band 80, 1974, Seite 1206CS, rechte Spalte, Zeilen 65 bis 66**
**Chemical Abstracts Band 85, Nr. 25, 20. Dezember 1976 Columbus, Ohio, USA M. De BOTTON « Reaction of chloro (ethoxymethyl) magnesium with some cyclanones II. Transformation of formylcyclanes into vinylogous aldehydes and ketones » Seite 510, linke Spalte, Abstract Nr. 192215y in Verbindung mit Chemical Abstracts Subject Index Band 85, 1976, Seite 1622Cs, rechte Spalte, Zeile 93 & Bull. Soc. Chim. Fr. Nr. 5-6, Teil 2, 1976, Seiten 849 to 856.**

(73) Patentinhaber : **CHEMISCHE WERKE HÜLS AG**
**Postfach 1320**
**D-4370 Marl 1 (DE)**

(72) Erfinder : **Burzin, Klaus, Dr.**
**Wellerfeldweg 164**
**D-4370 Marl (DE)**
Erfinder : **Otte, Werner, Dr.**
**Lipper Weg 195**
**D-4370 Marl (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 022 460 B1

Aliphatische Ether des Hydroximethylcyclododecans und ihre Verwendung zur Herstellung von Riechstoffkompositionen

Es wurde gefunden, daß aliphatische Ether des Hydroximethylcyclododecans der allgemeinen Formel

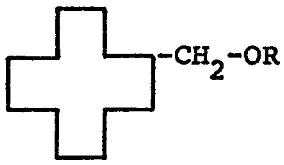

in der R ein geradkettiger oder verzweigter gesättigter oder ungesättigter Alkylrest mit 1 bis 4 Kohlenstoffatomen ist, neue Riechstoffe mit einer intensiven, nachhaltig wirkenden holzig-ambraartigen Geruchsnote darstellen, und daß sie eine sehr gute Kombinationsfähigkeit zu neuartigen Geruchsnuancen sowie ausgezeichnete Haftfestigkeit besitzen.

Verbindungen der Struktur

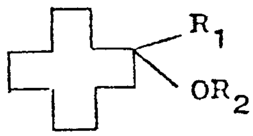

$R_1 = C_1$-$C_3$-Alkyl
$R_2 = CH_3$, $C_2H_5$

sind aus der DE-AS 21 52 016 bekannt. Der Geruch wird als holzig, z.B. zedrig bis ambraartig, tabakartig beschrieben. Diese Ether werden in 4 Stufen aus dem Cyclododecatetraen hergestellt.

Die Herstellung der erfindungsgemäßen Ether erfolgt gewöhnlich durch Veretherung des Hydroximethylcyclododecans. Dabei können die üblichen in der Literatur beschriebenen Veretherungsverfahren angewendet werden [s. Houben-Weyl, Methoden der organischen Chemie, Band VI/3, Sauerstoffverbindungen I, Teil 3, Seiten 10-137 (1965)].

Die Reinigung der Rohether erfolgt in der Regel durch fraktionierte Destillation. Die Entfernung letzter Reste nichtumgesetzter Alkohole ist auch durch andere Trennverfahren, wie z.B. Extraktions- oder Absorptionsverfahren möglich.

Das Hydroximethylcyclodecan läßt sich z.B. unter den Bedingungen einer Oxosynthese aus Cyclododecatrien-(1.5.9), Kohlenmonoxid und Wasserstoff in Gegenwart spezieller Katalysatoren nach bekannten Verfahren des Standes der Technik herstellen (DE-ASS 12 11 174 und 16 68 255, US-PS 33 54 229 sowie FR-PS 14 38 811).

Es ist überraschend, daß die erfindungsgemäßen aliphatischen Ether des großtechnisch gut zugänglichen Alkohols Hydroximethylcyclododecan sich durch einen intensiven typischen Holzgeruch mit Ambranote von großer Fülle auszeichnen und zur Herstellung von wertvollen Stoffkombinationen, insbesondere von Riechstoffkompositionen verwendet werden können.

Typische Vertreter der erfindungsgemäßen Ether sind der Cyclododecylmethyl-methyl-ether, der Cyclododecylmethylethyl-ether, der Cyclododecylmethyl-isopropyl-ether, der Cyclododecylmethyl-1-propenyl-ether und der Cyclododecylmethyl-allyl-ether. Unter diesen besitzen der Methyl- und der Ethylether die größte Bedeutung, da sie die geruchsintensivsten Verbindungen darstellen.

Mit anderen Stoffen, vornehmlich mit anderen Riechstoffen, können die erfindungsgemäßen Ether in den verschiedensten Mengenverhältnissen zu neuen Riechstoffkompositionen vermischt werden. Dabei beträgt der Anteil der beanspruchten Ether im allgemeinen bis zu 60, vorzugsweise 1 bis 50, Gewichtsprozent.

Die Riechstoffkompositionen können direkt als Parfüm oder zur Parfümierung von Kosmetika, wie z.B. Cremes, Seifen und Lotionen dienen. Eine weitere Verwendungsmöglichkeit ist die Geruchsverbesserung technischer Produkte, wie z.B. Wasch- und Reinigungsmittel, Desinfektionsmittel und Textilhilfsmittel.

Die nachfolgenden Beispiele sollen den Gegenstand der vorliegenden Erfindung näher erläutern, ohne ihn jedoch hierauf zu beschränken, da sich die übrigen aliphatischen Ether auf entsprechende Weise herstellen lassen und ebenfalls den typischen Holzgeruch mit Ambranote besitzen.

## Beispiel 1

Cyclododecylmethyl-methyl-ether

25 g (0,65 mol) Natriumamid wurden in 150 ml Xylol eingebracht und unter Rühren bis zum Sieden erhitzt. Innerhalb einer Stunde wurden 99 g (0,5 mol) Hydroximethylcyclododecan, gelöst in 750 ml Xylol,

in die siedende Suspension getropft. Zur Vervollständigung der Alkoholatbildung wurde weitere zwei Stunden am Rückfluß erhitzt. Danach wurden tropfenweise 44 g (0,35 mol) Dimethylsulfat hinzugefügt. Das Reaktionsgemisch wurde weitere vier Stunden rückfließend erhitzt und anschließend in eine Mischung aus Eis und 35 g Natriumhydroxid gegossen. Die organische Phase wurde mit Wasser gewaschen, mit $Na_2SO_4$ getrocknet und danach destilliert.

| | |
|---|---|
| Siedepunkt: | 87-90 °C bei 0,1 mbar |
| Ausbeute: | 94 % Rohether, 68 % reines Produkt |
| Brechungsindex $n_{20}^D$: | 1,475 1 |
| IR: | 1 380 $cm^{-1}$, 1 115 $cm^{-1}$ |
| NMR ($CCl_4$): | δ3,19 (S) ($CH_3$-O), δ3,10 (D) (—$CH_2$—O—) |

Der Cyclododecylmethyl-methyl-ether weist einen intensiven angenehmen Geruch nach Holz auf.

Beispiel 2

Cyclododecylmethyl-ethyl-ether

Der Cyclododecylmethyl-ethyl-ether wurde entsprechend den Angaben in Beispiel 1 durch Umsetzung von 99 g Hydroximethylcyclododecan mit 54 g (0,35 mol) Diethylsulfat gewonnen.

| | |
|---|---|
| Siedepunkt: | 105-110 °C bei 0,2 mbar |
| Ausbeute: | 92 % Rohether, 62 % reines Produkt |
| Brechungsindex $n_{20}^D$: | 1,472 0 |
| IR: | 1 380 $cm^{-1}$, 1 120 $cm^{-1}$ |
| NMR ($CCl_4$): | δ3,35 (Q) (O—$CH_2$—$CH_3$), δ3,15 D(—$CH_2$—O—$C_2H_5$) |

Der Cyclododecylmethyl-ethyl-ether besitzt einen angenehmen intensiven Holzgeruch.

Beispiel 3

Cyclododecylmethyl-isopropyl-ether

Nach den Angaben in Beispiel 1 wurden zunächst 99 g Hydroximethyl-cyclododecan in das Natriumalkoholat übergeführt. In die siedende Suspension wurden 74 g (0,6 mol) 2-Brompropan zugetropft und anschließend 30 Stunden unter Rückfluß erhitzt. Das Reaktionsgemisch wurde in Wasser gegossen, die organische Phase mit Wasser gewaschen, über $Na_2SO_4$ getrocknet und anschließend destilliert.

| | |
|---|---|
| Siedepunkt: | 110-112 °C bei 0,3 mbar |
| Ausbeute: | 89 % Rohether, 79 % reines Produkt |
| Brechungsindex $n_{20}^D$: | 1,481 4 |
| IR: | 1 370 $cm^{-1}$, 1 357 $cm^{-1}$, 1 120 $cm^{-1}$ |
| NMR ($CDCl_3$): | δ3,40 (Hept.) 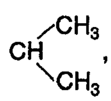, δ3,20 (D) (—$CH_2$—O—$C_3H_7$) |

Der Cyclododecylmethyl-isopropyl-ether weist einen Geruch nach Holz auf.

Beispiel 4

Cyclododecylmethyl-allyl-ether

Der Cyclododecylmethyl-allyl-ether wurde entsprechend den Angaben in Beispiel 1 und Beispiel 3 hergestellt, wobei anstelle des 2 Brompropans 73 g (0,6 mol) 1-Brompropen-(2) eingesetzt wurden. Das Reaktionsgemisch wurde 24 Stunden rückfließend erhitzt, anschließend in Wasser gegossen, mit Wasser gewaschen, über $Na_2SO_4$ getrocknet und destilliert.

| | |
|---|---|
| Siedepunkt: | 115-118 °C bei 0,2 mbar |
| Ausbeute: | 88 % Rohether, 79 % reines Produkt |
| Brechungsindex $n_{20}^D$: | 1,481 2 |
| IR: | 1 640 $cm^{-1}$, 1 100 $cm^{-1}$, 990 $cm^{-1}$, 910 $cm^{-1}$ |

NMR ($CCl_4$): δ3,82 (D) ($O—\underline{CH_2}—CH = CH_2$), δ3,15 (D) ($—\underline{CH_2}—O—CH_2—CH = CH_2$)

Der Cyclododecylmethyl-allyl-ether besitzt einen angenehmen Holzgeruch.

Nachfolgend werden Beispiele für Riechstoffkompositionen aufgeführt, in denen die erfindungsgemäßen Substanzen enthalten sind :

| Beispiel 5 (Holzbase) | Gew.-Teile |
|---|---|
| Cyclododecylmethyl-methyl-ether | 250 |
| Cyclododecylmethyl-ethyl-ether | 200 |
| Cyclododecylmethyl-allyl-ether | 50 |
| Oryclon | 100 |
| Vetiverylacetat | 100 |
| Isoraldein 70 | 50 |
| Guajylacetat | 50 |
| Zedernketon | 100 |
| Phenylethylalkohol | 50 |
| Cumarin | 50 |
| | 1 000 Gew.-Teile |

| Beispiel 6 (Seifenparfüm) | Gew.-Teile |
|---|---|
| Cyclododecylmethyl-methyl-ether | 200 |
| Cyclododecylmethyl-ethyl-ether | 75 |
| Cyclododecylmethyl-isopropyl-ether | 75 |
| Methylanthralinat | 100 |
| Bergamottöl | 70 |
| Tolubalsam | 25 |
| Indol | 5 |
| Limonen | 450 |
| | 1 000 Gew.-Teile |

**Ansprüche**

1. Aliphatische Ether des Hydroximethylcyclododecans der allgemeinen Formel

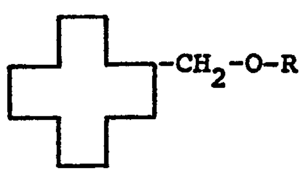

in der R ein geradkettiger oder verzweigter gesättigter oder ungesättigter Alkylrest mit 1 bis 4 Kohlenstoffatomen ist.

2. Cyclododecylmethyl-methyl-ether.
3. Cyclododecylmethyl-ethyl-ether.
4. Cyclododecylmethyl-isopropyl-ether.
5. Cyclododecylmethyl-1-propenyl-ether.
6. Cyclododecylmethyl-allyl-ether.
7. Riechstoffkomposition, gekennzeichnet durch einen Gehalt an einer Verbindung nach Anspruch 1.
8. Verwendung der Verbindungen nach Anspruch 1 zur Herstellung von Riechstoffkompositionen.

**Claims**

1. Aliphatic ethers of hydroxymethylcyclododecane of the general formula

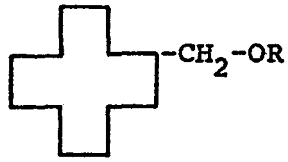

where R is a straight-chain or branched, saturated or unsaturated aliphatic hydrocarbon radical of 1 to 4 carbon atoms.

2. Cyclododecylmethyl methyl ether.
3. Cyclododecylmethyl ethyl ether.
4. Cyclododecylmethyl isopropyl ether.
5. Cyclododecylmethyl 1-propenyl ether.
6. Cyclododecylmethyl allyl ether.
7. Odoriferous compositions characterised by a content of a compound according to claim 1.
8. The use of compounds according to claim 1 in the preparation of odoriferous compositions.

**Revendications**

1. Ethers aliphatiques de l'hydroxyméthyl-cyclododécane, de la formule générale

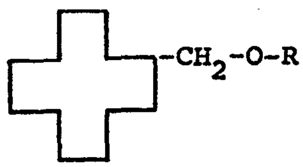

dans laquelle R est un reste alkyle à chaîne droite ou ramifié, saturé ou non saturé, qui comporte de 1 à 4 atomes de carbone.

2. L'éther cyclododécylméthyl-méthylique.
3. L'éther cyclododécylméthyl-éthylique.
4. L'éther cyclododécylméthyl-isopropylique.
5. L'éther cyclododécylméthyl-1-propénylique.
6. L'éther cyclododécylméthyl-allylique.
7. Une composition odorante, caractérisée par le fait qu'elle renferme un composé suivant la revendication 1.
8. L'utilisation, pour la préparation de compositions odorantes, des composés suivant la revendication 1.